(19) Europäisches Patentamt
European Patent Office
Office européen des brevets

(11) **EP 3 081 143 A1**

(12) **EUROPEAN PATENT APPLICATION**
published in accordance with Art. 153(4) EPC

(43) Date of publication:
**19.10.2016 Bulletin 2016/42**

(51) Int Cl.:
***A61B 1/00*** (2006.01)     ***A61B 1/06*** (2006.01)

(21) Application number: **15803237.5**

(22) Date of filing: **02.03.2015**

(86) International application number:
**PCT/JP2015/056052**

(87) International publication number:
**WO 2015/186383 (10.12.2015 Gazette 2015/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **02.06.2014 JP 2014114356**

(71) Applicant: **Olympus Corporation**
**Tokyo 192-8507 (JP)**

(72) Inventor: **TAKEI, Shunji**
**Tokyo 192-8507 (JP)**

(74) Representative: **Schicker, Silvia**
**Wuesthoff & Wuesthoff**
**Patentanwälte PartG mbB**
**Schweigerstraße 2**
**81541 München (DE)**

(54) **ILLUMINATION DEVICE**

(57) A lighting apparatus includes: a light source unit configured to emit first light and second light by sequentially switching between the first light and the second light; a light-receiving unit configured to receive the first light with first light-receiving sensitivity and output a first light detection signal and receive the second light with second light-receiving sensitivity and output a second light detection signal; a multiplication unit configured to generate a first multiplication signal and a second multiplication signal, where the first multiplication signal is obtained by multiplying the first light detection signal by a coefficient set according to the first light-receiving sensitivity and the second multiplication signal is obtained by multiplying the second light detection signal by a coefficient set according to the second light-receiving sensitivity; an integration unit configured to integrate at least one of the first and second multiplication signals for a predetermined period; a judging unit configured to judge whether or not integration results exceed a threshold for judging a condition of the light source unit; and a control unit configured to control at least one of the first and second light sources based on a judgement result.

**FIG. 1**

EP 3 081 143 A1

**Description**

Technical Field

The present invention relates to a lighting apparatus, and more particularly to a lighting apparatus configured to emit light by switching among plural wavelength bands.

Background Art

In relation to endoscopes in a medical field, various techniques for reducing a diameter of an insertion portion inserted into a body cavity of a subject have been proposed to reduce burdens on the subject. As an example of such techniques, a scanning endoscope and a scanning endoscope system equipped with the scanning endoscope are known, where the scanning endoscope does not have a solid-state image pickup device in a portion corresponding to the above-mentioned insertion portion.

Specifically, the scanning endoscope system described above is configured, for example, to two-dimensionally scan an object in a preset scanning pattern, by swinging a distal end portion of an illumination fiber for guiding light emitted from a light source, receive return light from the object using light-receiving fibers placed around the illumination fiber, and generate an image of the object based on the return light received by the light-receiving fibers. As an endoscope having a configuration similar to such a scanning endoscope system, for example, a scanning endoscope apparatus disclosed in Japanese Patent No. 5363688 is known.

Now, the scanning endoscope system is generally configured to scan the object by irradiating the object with a laser beam. Therefore, it is desirable that the scanning endoscope system, for example, has a configuration for reliably detecting whether or not there is anything wrong with the light source, to prevent the object from being irradiated with excessively strong light.

However, Japanese Patent No. 5363688 does not make a particular disclosure or the like about a configuration for reliably detecting whether or not there is anything wrong with the light source.

The present invention has been made in view of the above circumstances and has an object to provide a lighting apparatus capable of reliably detecting whether or not there is anything wrong with a light source.

Disclosure of Invention

Means for Solving the Problem

A lighting apparatus according to one aspect of the present invention includes a light source unit provided with a first light source for emitting first light and a second light source for emitting second light in a wavelength band different from the first light and configured to emit the first light and the second light by sequentially switching between the first light and the second light; a light-receiving unit configured to receive the first light with first light-receiving sensitivity and output a first light detection signal and receive the second light with second light-receiving sensitivity different from the first light-receiving sensitivity and output a second light detection signal; a multiplication unit configured to generate a first multiplication signal and a second multiplication signal, where the first multiplication signal is obtained by multiplying the first light detection signal by a coefficient set according to the first light-receiving sensitivity and the second multiplication signal is obtained by multiplying the second light detection signal by a coefficient set according to the second light-receiving sensitivity; an integration unit configured to integrate at least one of the first multiplication signal and the second multiplication signal for a predetermined period, the first and second multiplication signals being generated by the multiplication unit; a judging unit configured to judge whether or not integration results produced by the integration unit exceed a threshold for judging a condition of the light source unit; and a control unit configured to control at least one of the first light source and the second light source based on a judgement result produced by the judging unit.

A lighting apparatus according to one aspect of the present invention includes a light source unit provided with a first light source for emitting first light and a second light source for emitting second light in a wavelength band different from the first light and configured to emit the first light and the second light by sequentially switching between the first light and the second light; a light-receiving unit configured to receive the first light with first light-receiving sensitivity and output a first light detection signal and receive the second light with second light-receiving sensitivity different from the first light-receiving sensitivity and output a second light detection signal; an optical filter placed at a predetermined position on an optical path between the light source unit and the light-receiving unit and configured to cause the first light emitted from the light source unit to be transmitted at first transmittance corresponding to an inverse of the first light-receiving sensitivity and cause the second light emitted from the light source unit to be transmitted at second transmittance corresponding to an inverse of the second light-receiving sensitivity; an integration unit configured to integrate at least one of the first light detection signal and the second light detection signal outputted from the light-receiving unit for a predetermined period; a judging unit configured to judge whether or not integration results produced by the integration unit exceed a threshold for judging a condition of the light source unit; and a control unit configured to control at least one of the first light source and the second light source based on a judgement result produced by the judging unit.

Brief Description of the Drawings

Fig. 1 is a diagram showing a configuration of principal part of an endoscope system according to a first embodiment;
Fig. 2 is a diagram showing an example of a relationship between wavelength bands of R light, G light, and B light and spectral sensitivity characteristics of a photodiode;
Fig. 3 is a diagram showing an example of driving currents supplied to respective light sources;
Fig. 4 is a diagram showing an example of pulsed lights emitted from respective light sources;
Fig. 5 is a diagram showing an example of a pulse signal outputted from a photodiode which has received pulsed lights with intensities such as shown in Fig. 4;
Fig. 6 is a diagram showing an example of time variation of integrated values;
Fig. 7 is a diagram showing an example of time variation of a total value;
Fig. 8 is a diagram showing an example of time variation in a count value of elapsed time;
Fig. 9 is a diagram showing an example of time variation in count values of delay times;
Fig. 10 is a diagram showing a configuration of principal part of an endoscope system according to a second embodiment;
Fig. 11 is a diagram showing an example of relationships among wavelength bands of R light, G light, and B light, spectral sensitivity characteristics of a photodiode, and transmission characteristics of an optical filter;
Fig. 12 is a diagram showing an example of pulsed lights which have passed through an optical filter having transmission characteristics such as shown in Fig. 11; and
Fig. 13 is a diagram showing an example of a pulse signal outputted from a photodiode which has received pulsed lights with intensities such as shown in Fig. 12.

Best Mode for Carrying Out the Invention

Embodiments of the present invention will be described below with reference to the drawings.

(First Embodiment)

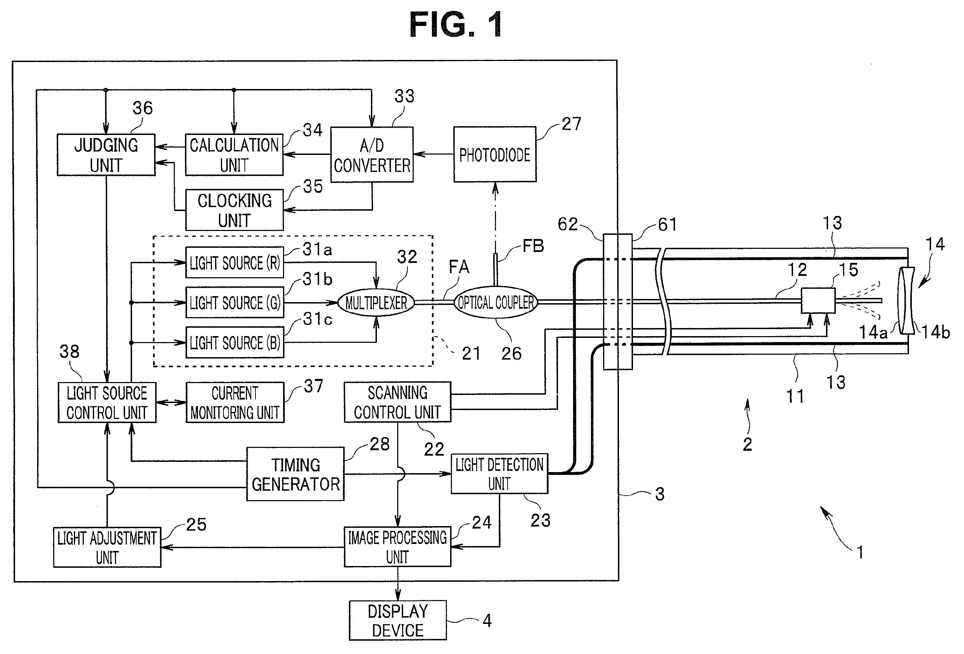

Figs. 1 to 9 relate to a first embodiment of the present invention. Fig. 1 is a diagram showing a configuration of principal part of an endoscope system according to the first embodiment.
For example, as shown in Fig. 1, the endoscope system 1 includes a scanning endoscope 2 inserted into a body cavity of a subject, a main apparatus 3 connectable with the endoscope 2, and a display device 4 connected to the main apparatus 3.
The endoscope 2 includes an insertion portion 11 provided with an elongated shape and flexibility and configured to be insertable into the body cavity of the subject.
A proximal end portion of the insertion portion 11 is provided with a connector unit 61 used to detachably connect the endoscope 2 to a connector jack unit 62 of the main apparatus 3.
An illumination fiber 12 configured to lead light supplied from a light source unit 21 of the main apparatus 3 to a condensing optical system 14 as well as light-receiving fibers 13 configured to receive return light from the object and lead the return light to a light detection unit 23 of the main apparatus 3 are each passed through a part from the proximal end portion to a distal end portion inside the insertion portion 11.
An incident end portion including a light incidence surface of the illumination fiber 12 is placed on an optical coupler 26 provided inside the main apparatus 3. Also, an emitting end portion including a light emission surface of the illumination fiber 12 is placed near a light incidence surface of a lens 14a provided in a distal end portion of the insertion portion 11.
An incident end portion including a light incidence surface of the light-receiving fibers 13 is fixedly placed around a light emission surface of a lens 14b on a distal end face of the distal end portion of the insertion portion 11. Also, an emitting end portion including a light emission surface of the light-receiving fibers 13 is placed near the light detection unit 23 provided inside the main apparatus 3.
The condensing optical system 14 includes the lens 14a which the light passing through the light emission surface of the illumination fiber 12 enters and the lens 14b configured to emit the light passing through the lens 14a to the object.

An actuator unit 15 configured to be able to swing an emitting end portion of the illumination fiber 12 by being driven based on a drive signal supplied from a scanning control unit 22 of the main apparatus 3 is provided in a mid-portion of the illumination fiber 12 on the side of the distal end portion of the insertion portion 11.

The actuator unit 15 is made up, for example, of a first actuator and a second actuator, where the first actuator includes one or more piezoelectric elements capable of swinging the emitting end portion of the illumination fiber 12 along a first direction by being driven based on a drive signal supplied from the scanning control unit 22 of the main apparatus 3 and the second actuator includes one or more piezoelectric elements capable of swinging the emitting end portion along a second direction orthogonal to the first direction by being driven based on a drive signal supplied from the scanning control unit 22 of the main apparatus 3.

The main apparatus 3 includes the light source unit 21 configured to supply illuminating light used to illuminate the object to an optical fiber FA, the optical coupler 26 configured to cause light to enter a light incidence surface of an optical fiber FB by branching light going from a light emission surface of the optical fiber FA to the light incidence surface of the illumination fiber 12, and a photodiode 27 configured to receive light emitted from a light emission surface of the optical fiber FB and generate and output an electrical signal according to intensity of the received light. Also, the main apparatus 3 includes an A/D converter 33, a calculation unit 34, a clocking unit 35, a judging unit 36, a current monitoring unit 37, and a light source control unit 38.

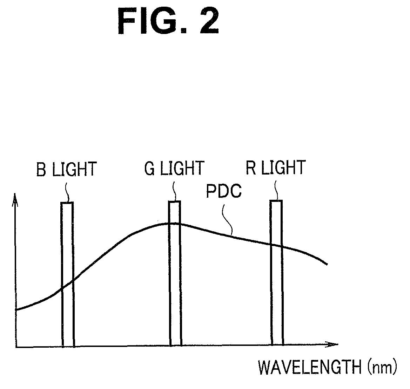

The light source unit 21 includes a light source 31a, a light source 31b, a light source 31c and a multiplexer 32. Fig. 2 is a diagram showing an example of a relationship between wavelength bands of R light, G light, and B light and spectral sensitivity characteristics of a photodiode.

The light source 31a is made up, for example, of a laser light source and configured to switch between an ON state and OFF state under control of the light source control unit 38. Also, the light source 31a is configured to emit red light (hereinafter also referred to as R light) having a wavelength band such as shown in Fig. 2 to the multiplexer 32 upon entering an ON state under the control of the light source control unit 38.

The light source 31b is made up, for example, of a laser light source and configured to switch between an ON state and OFF state under the control of the light source control unit 38. Also, the light source 31b is configured to emit green light (hereinafter also referred to as G light) having a wavelength band such as shown in Fig. 2 to the multiplexer 32 upon entering an ON state under the control of the light source control unit 38.

The light source 31c is made up, for example, of a laser light source and configured to switch between an ON state and OFF state under the control of the light source control unit 38. Also, the light source 31c is configured to emit blue light (hereinafter also referred to as B light) having a wavelength band such as shown in Fig. 2 to the multiplexer 32 upon entering an ON state under the control of the light source control unit 38.

The multiplexer 32 is configured to be able to combine the R light emitted from the light source 31a, the G light emitted from the light source 31b, and the B light emitted from the light source 31 c and supply resulting light to a light incidence surface of the optical fiber FA.

The A/D converter 33 is configured to convert an electrical signal outputted from the photodiode 27 into a digital pulse signal and output the pulse signal to the calculation unit 34 and clocking unit 35.

The calculation unit 34 is made up, for example, of an arithmetic processing circuit equipped with an integrator. Also, the calculation unit 34 is configured to acquire an integrated value Sp by integrating output values of the pulse signal outputted from the A/D converter 33, calculate a corrected integrated value CSp by multiplying the acquired integrated value Sp by a correction coefficient C, and output the calculated integrated value CSp to the judging unit 36. Also, the calculation unit 34 is configured to acquire a total value Sc, which is a sum total of the integrated values CSp calculated within a base period BT and output the acquired total value Sc to the judging unit 36. Note that according to the present embodiment, preferably the above-mentioned base period BT is set to 0.25 seconds.

The clocking unit 35 is made up, for example, of the clocking circuit equipped with a timer and the like. Also, the clocking unit 35 is configured to acquire a count value of an elapsed time ET starting from a time point at which the pulse signal from the A/D converter 33 starts being inputted, i.e., a time point at which it is detected that an output value of the pulse signal becomes larger than 0 and output the acquired count value of the elapsed time ET to the judging unit 36. Also, the clocking unit 35 is configured to output the count value of the elapsed time ET to the judging unit 36 and reset the count value to 0 at a time point when the pulse signal from the A/D converter 33 stops being inputted, i.e., at a time point when it is detected that the output value of the pulse signal becomes 0.

The judging unit 36 is made up, for example, of a judging circuit. Also, the judging unit 36 is configured to compare the integrated value Sp outputted from the calculation unit 34 with a threshold TH1 described later, compare the total value Sc outputted from the calculation unit 34 with a threshold TH2 described later, compare the count value of the elapsed time ET outputted from the clocking unit 35 with a threshold TH3 described later, thereby perform a predetermined judgment process (described later), and output a judgement result obtained by the predetermined judgment process to the light source control unit 38.

The current monitoring unit 37 is made up, for example, of an ammeter. Also, the current monitoring unit 37 is configured to measure amperage of each of driving currents supplied to the light sources 31 a, 31 b, and 31 c, from the light source control unit 38, and output the measured amperages to the light source control unit 38.

The light source control unit 38 is made up, for example, of a CPU or a control circuit. Also, the light source control unit 38 is configured to perform an operation intended to cause pulsed R light, G light, and B light to be emitted in a predetermined order every predetermined time $\tau$.

The light source control unit 38 is configured to continue control in order to cause pulsed R light, G light, and B light to be emitted in a predetermined order every predetermined time $\tau$ when it is detected, based on the judgement result outputted from the judging unit 36, that each of the light sources 31a, 31b, and 31c is operating normally. Also, when it is detected, based on the judgement result outputted from the judging unit 36, that something is wrong with at least one of the light sources 31 a, 31 b, and 31 c, the light source control unit 38 is configured to perform an operation for turning off all the light sources 31 a, 31 b, and 31c and thereby stop emission of the R light, G light, and B light.

The light source control unit 38 is configured to set the amperages of the driving currents supplied to the light sources 31 a, 31 b, and 31 c based on the amperage outputted from the current monitoring unit 37 and a light adjustment value outputted from a light adjustment unit 25 described later.

The photodiode 27 has, for example, of spectral sensitivity characteristics such as represented by line segment PDC in Fig. 2. Specifically, the photodiode 27 has spectral sensitivity characteristics which satisfy a relationship of $Qb < Qr < Qg$, where $Qr$ denotes light-receiving sensitivity in a wavelength band of the R light, $Qg$ denotes light-receiving sensitivity in a wavelength band of the G light, and $Qb$ denotes light-receiving sensitivity in a wavelength band of the B light.

On the other hand, the main apparatus 3 includes the scanning control unit 22, the light detection unit 23, the light detection unit 23, an image processing unit 24, the light adjustment unit 25, and a timing generator 28.

The scanning control unit 22 is made up, for example, of a signal generator. Also, the scanning control unit 22 is configured to generate a drive signal intended to cause the emitting end portion of the illumination fiber 12 to swing in a predetermined scanning pattern such as a spiral pattern or a Lissajous pattern and output the generated drive signal to the actuator unit 15 and image processing unit 24.

The light detection unit 23 is made up, for example, of an avalanche photodiode, a signal amplifier, and an A/D converter. Also, the light detection unit 23 is configured to detect return light emitted from the light emission surface of the light-receiving fibers 13, generate a signal which represents a luminance value according to intensity of the detected return light, and output the generated signal to the image processing unit 24.

The image processing unit 24 is made up, for example, of an image processing circuit such as an AGC (automatic gain control) circuit and gamma correction circuit. Also, the image processing unit 24 is configured to detect a scanning pattern of the object based on the drive signal outputted from the scanning control unit 22, maps luminance values represented by the signal outputted from the light detection unit 23 to pixels at positions corresponding to the detected scanning pattern, perform an interpolation process to interpolate a luminance value of each pixel left out of mapping, and thereby generate an image of the object. Also, the image processing unit 24 is configured to output the image of the object generated as described earlier to the light adjustment unit 25. Also, the image processing unit 24 is configured to apply processes such as gain adjustment and gamma correction to the object image generated as described earlier, thereby generating a display image, and output the generated display image to the display device 4.

The light adjustment unit 25 is made up, for example, of a light-adjusting circuit. Also, the light adjustment unit 25 is configured, for example, to calculate an average value of the luminance values of image outputted from the image processing unit 24, generate a light adjustment signal intended to bring a difference between the calculated average value of the luminance values and a predetermined target value of brightness close to 0, and output the generated light adjustment signal to the light source control unit 38.

The timing generator 28 is configured to generate and output a synchronizing signal used to synchronize operation timing of the light detection unit 23, A/D converter 33, calculation unit 34, and judging unit 36 with emission timing of the R light, G light, and B light in control performed by the light source control unit 38. Note that it is assumed that the above-mentioned synchronizing signal may be generated, being provided, for example, with a waveform or the like which allows emission timing of the R light, emission timing of the G light, and emission timing of the B light to be identified individually.

Next, operation of the present embodiment will be described.

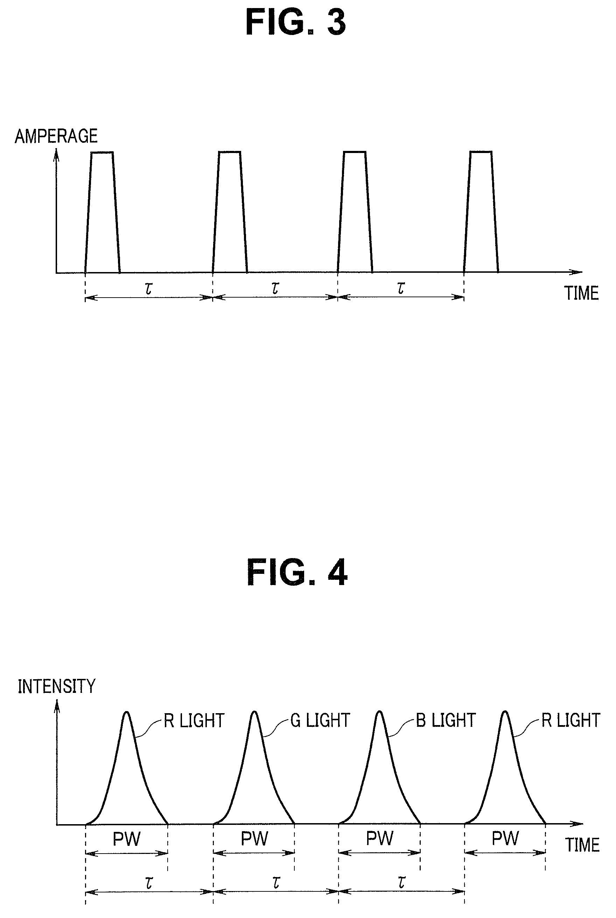

After the main apparatus 3 is powered on, the light source control unit 38 operates, being timed with a synchronizing signal from the timing generator 28, and thereby performs an operation of switching a destination of a pulsed driving current such as shown in Fig. 3 every predetermined time $\tau$ in the order: light sources 31 a -> 31b -> 31c -> 31a -> ... Then, the operation of the light source control unit 38 causes the R light, G light, and B light, which are pulsed lights having a pulse width PW such as shown in Fig. 4, to be emitted from the light emission surface of the optical fiber FB in this order every predetermined time $\tau$. Fig. 3 is a diagram showing an example of driving currents supplied to respective light sources. Fig. 4 is a diagram showing an example of pulsed lights emitted from respective light sources.

The photodiode 27 receives light emitted sequentially from the light emission surface of the optical fiber FB generates an electrical signal according to the intensity of the received light, and outputs the electrical signal to the A/D converter 33.

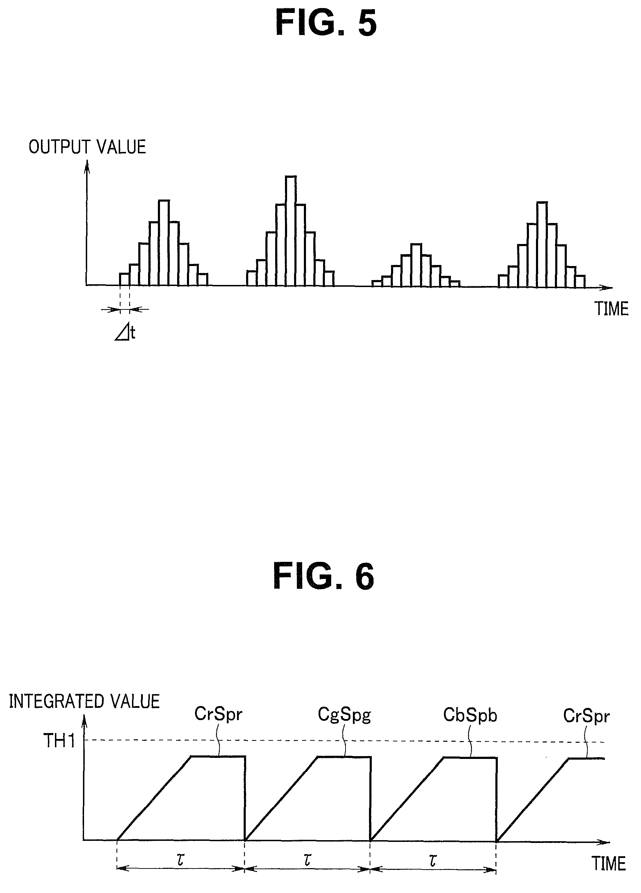

The A/D converter 33 operates, being timed with a synchronizing signal from the timing generator 28, thereby converts the electrical signal outputted from the photodiode 27 into a pulse signal every sampling time $\Delta t$, which is shorter in duration than the pulse width PW, and outputs the pulse signal to the calculation unit 34 and clocking unit 35. Then, the operation of the A/D converter 33 causes a pulse signal with output values such as shown in Fig. 5 to be outputted sequentially to the calculation unit 34 and clocking unit 35, as a pulse signal according to intensities of the R light, G light, and B light received sequentially by the photodiode 27. Fig. 5 is a diagram showing an example of a pulse signal outputted from a photodiode which has received pulsed lights with intensities such as shown in Fig. 4.

Based on the synchronizing signal from the timing generator 28, the calculation unit 34 acquires an integrated value Spr by integrating output values of a pulse signal Sdr inputted being timed with the emission timing of the R light, calculates a corrected integrated value CrSpr by multiplying the acquired integrated value Spr by a correction coefficient Cr, and outputs the integrated value CrSpr to the judging unit 36.

Based on the synchronizing signal from the timing generator 28, the calculation unit 34 acquires an integrated value Spg by integrating output values of a pulse signal Sdg inputted being timed with the emission timing of the G light, calculates a corrected integrated value CgSpg by multiplying the acquired integrated value Spg by a correction coefficient Cg, and outputs the integrated value CgSpg to the judging unit 36.

Based on the synchronizing signal from the timing generator 28, the calculation unit 34 acquires an integrated value Spb by integrating output values of a pulse signal Sdb inputted being timed with the emission timing of the B light, calculates a corrected integrated value CbSpb by multiplying the acquired integrated value Spb by a correction coefficient Cb, and outputs the integrated value CbSpb to the judging unit 36.

That is, with the above-mentioned calculation unit 34, a period during which the integrated value CrSpr is calculated and outputted to the judging unit 36, a period during which the integrated value CgSpg is calculated and outputted to the judging unit 36, and a period during which the integrated value CbSpb is calculated and outputted to the judging unit 36 are switched in this order every predetermined time $\tau$.

Note that values of the correction coefficients Cr, Cg, and Cb described above are each set to satisfy a relationship of $Cr \times Qr = Cg \times Qg = Cb \times Qb$ with the light-receiving sensitivities Qr, Qg, and Qb of the photodiode 27. Specifically, for example, the correction coefficients Cr, Cg, and Cb can be set to satisfy $Cr = 1/Qr$, $Cg = 1/Qg$, and $Cb = 1/Qb$.

Also, the calculation unit 34 operates, being timed with the synchronizing signal from the timing generator 28, thereby acquires a total value SA, which is a sum total of the integrated values CrSpr, CgSpg, and CbSpb calculated within the base period BT longer in duration than the predetermined time $\tau$, and outputs the total value SA to the judging unit 36. Note that each time a new integrated value is calculated, the calculation unit 34 performs a process of acquiring the total value SA by overwriting the oldest of all the integrated values calculated within the base period BT with the new integrated value. With this process, the total value SA outputted to the judging unit 36 is updated every predetermined time $\tau$.

The clocking unit 35 operates, being timed with a synchronizing signal from the timing generator 28, thereby acquires the count value of the elapsed time ET, and outputs the acquired count value of elapsed time ET to the judging unit 36.

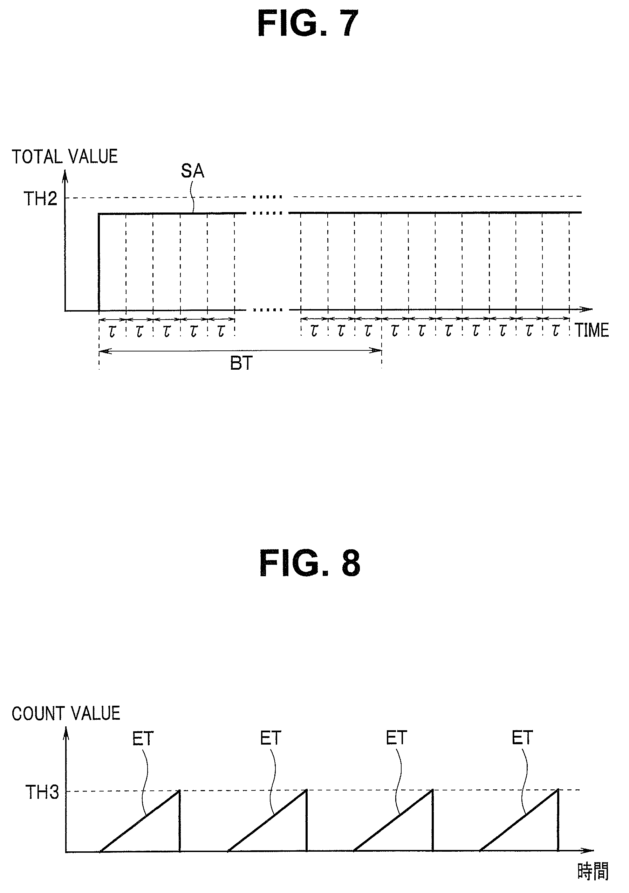

The judging unit 36 detects the integrated values CrSpr, CgSpg, and CbSpb outputted from the calculation unit 34, for example, as shown in Fig. 6. Also, the judging unit 36 detects the total value SA outputted from the calculation unit 34, for example, as shown in Fig. 7. Also, the judging unit 36 detects the count value of the elapsed time ET outputted from the clocking unit 35, for example, as shown in Fig. 8. Fig. 6 is a diagram showing an example of time variation of integrated values. Fig. 7 is a diagram showing an example of time variation of a total value. Fig. 8 is a diagram showing an example of time variation in a count value of elapsed time.

Then, when it is detected that all the integrated values CrSpr, CgSpg, and CbSpb outputted from the calculation unit 34 are equal to or smaller than the threshold TH1, that the total value SA outputted from the calculation unit 34 is equal to or smaller than the threshold TH2, and that the count value of the elapsed time ET outputted from the clocking unit 35 is equal to or smaller than the threshold TH3, the judging unit 36 acquires a judgement result indicating that each of the light sources 31 a, 31 b, and 31 c is operating normally and outputs the acquired judgement result to the light source control unit 38.

Also, when it is detected that any of the integrated values CrSpr, CgSpg, and CbSpb outputted from the calculation unit 34 exceeds the threshold TH1, that the total value SA outputted from the calculation unit 34 exceeds the threshold TH2, or that the count value of the elapsed time ET outputted from the clocking unit 35 exceeds the threshold TH3, the judging unit 36 acquires a judgement result indicating that something is wrong with at least one of the light sources 31a, 31b, and 31c, and outputs the acquired judgement result to the light source control unit 38.

Here, each of the threshold TH1 and threshold TH2 is set based on a standard value of AEL (accessible emission limit) stipulated in IEC 60825, which is an international standard prescribing safety standards for laser products. A concrete method for setting the thresholds TH1 and threshold TH2 will be described below.

According to the present embodiment, if, for example, a condition set forth in Expression (1) below regarding a threshold TH1r for the R light is not satisfied, it is detected that something is wrong with the light source 31a.

$$\int_{t_0}^{t} Svr(t) \cdot Cr dt < TH1r \quad \cdots \quad (1)$$

Here, t0 is start point of the time at which light is incident upon the photodiode 27, and Svr(t) is a voltage value of an electrical signal outputted from the photodiode 27 when R light with intensity Pir(t) is received.

On the other hand, if an intensity value (amount of energy) corresponding to the standard value of AEL is VS, the intensity Pir(t) of the R light incident upon the photodiode 27 has to satisfy a condition set forth in Expression (2) below. Note that the intensity value VS is stipulated to be constant within a visible range of 400 nm to 700 nm. That is, according to the present embodiment, upper limit values of emission intensities of the R light, G light, and B light emitted as pulsed light from the respective light sources of the light source unit 21 are stipulated to be less than the intensity value VS corresponding to the standard level of AEL.

$$\int_{t_0}^{t} Pir(t) dt < VS \quad \cdots \quad (2)$$

When a relationship of Svr(t) = Qr x Pir(t) holds, Expression (2) above can be transformed into Expression (3) below.

$$\int_{t_0}^{t} Svr(t) \cdot Cr dt < VS \cdot Qr \cdot Cr \quad \cdots \quad (3)$$

Then, based on the values on the right sides of Expressions (1) and (3), the threshold TH1r has to be set so as to satisfy Expression (4) below.

$$TH1r < VS \cdot Qr \cdot Cr \quad \cdots \quad (4)$$

If Cr x Qr = Cg x Qg = Cb x Qb = G, the threshold TH1r can be applied, as a common threshold TH1, to the R light, G light, and B light and Expression (4) above can be rewritten as Expression (5) below. Thus, the threshold TH1 according to the present embodiment has to be set so as to satisfy the relationship of Expression (5) below.

$$TH1 < VS \cdot G \quad \cdots \quad (5)$$

On the other hand, according to the present embodiment, if, for example, a condition set forth in Expression (6) below regarding the threshold TH2 is not satisfied, it is detected that something is wrong with at least one of the light sources 31a, 31b, and 31c.

$$\int_{t_0}^{T} Svr(t) \cdot Cr dt + \int_{t_0}^{T} Svg(t) \cdot Cg dt + \int_{t_0}^{T} Svb(t) \cdot Cb dt < TH2 \quad \cdots \quad (6)$$

Here, Svg(t) is a voltage value of an electrical signal outputted from the photodiode 27 when G light with intensity Pig(t) is received and Svb(t) is a voltage value of an electrical signal outputted from the photodiode 27 when B light with intensity Pib(t) is received. Therefore, Expression (6) above can be rewritten as Expression (7) below.

$$\int_{t_0}^{T} Qr \cdot Pir(t) \cdot Cr dt + \int_{t_0}^{T} Qg \cdot Pig(t) \cdot Cg dt$$
$$+ \int_{t_0}^{T} Qb \cdot Pib(t) \cdot Cb dt < TH2 \quad \cdots \quad (7)$$

Also, if Cr x Qr = Cg x Qg = Cb x Qb = G, Expression (7) above can be rewritten as Expression (8) below.

$$\int_{t_0}^{T} Pir(t)dt + \int_{t_0}^{T} Pig(t)dt + \int_{t_0}^{T} Pib(t)dt < TH2/G \quad \cdots \quad (8)$$

On the other hand, if an intensity value (amount of energy) corresponding to the standard value of AEL is VS, and if the total number of times the R light, G light, and B light are received by the photodiode 27 within a base period T is N, the intensities Pir(t), Pig(t), and Pib(t), of the R light, G light, and B light incident upon the photodiode 27 have to satisfy a condition set forth in Expression (9) below.

$$\int_{t_0}^{T} Pir(t)dt + \int_{t_0}^{T} Pig(t)dt + \int_{t_0}^{T} Pib(t)dt < VS \cdot N \quad \cdots \quad (9)$$

Thus, based on the values on the right sides of Expressions (8) and (9), the threshold TH2 according to the present embodiment has to be set so as to satisfy Expression (10) below.

$$TH2 < VS \cdot N \cdot G \quad \cdots \quad (10)$$

Calculation results obtained by performing calculations shown on the left sides of Expressions (1) to (3) are updated by the calculation unit 34 according to the present embodiment every predetermined time $\tau$. Also, the judging unit 36 according to the present embodiment compares the integrated values CrSpr, CgSpg, and CbSpb outputted from the calculation unit 34 with the threshold TH1 set to satisfy the relationship of Expression (5) above and thereby detects whether or not there is any light source which emits pulsed light whose intensity exceeds proper intensity controlled by the light source control unit 38.

Calculation results obtained by performing calculations shown on the left sides of Expressions (7) to (9) are updated by the calculation unit 34 according to the present embodiment every base time BT. Also, the judging unit 36 according to the present embodiment compares the total value SA outputted from the calculation unit 34 with the threshold TH2 set to satisfy the relationship of Expression (10) above and thereby detects whether or not there is any light source which emits pulsed light whose intensity exceeds proper intensity controlled by the light source control unit 38.

Also, the judging unit 36 according to the present embodiment compares the count value of the elapsed time ET outputted from the clocking unit 35 with the threshold TH3 and thereby detects whether or not there is any light source which exhibits disturbances in a waveform of pulsed light obtained by modulating a driving current supplied from the light source control unit 38.

Note that according to the present embodiment, for example, the judging unit 36 may count the number of input times Y from a time point at which the pulse signal from the A/D converter 33 starts being inputted to a time point at which the pulse signal from the A/D converter 33 stops being inputted, compare a value obtained by multiplying the number of input times Y by the sampling time $\Delta t$ with the threshold TH3, and thereby detect whether or not there is any light source which exhibits disturbances in the waveform of the pulsed light obtained by modulating the driving current supplied from the light source control unit 38.

When it is detected, based on the judgement result outputted from the judging unit 36, that each of the light sources 31a, 31b, and 31c is operating normally, the light source control unit 38 continues the operation of switching the destination of the pulsed driving current shown in Fig. 3 every predetermined time $\tau$ in the order: light sources 31a -> 31b -> 31c -> 31a -> ...

Also, when it is detected, based on the judgement result outputted from the judging unit 36, that something is wrong with at least one of the light sources 31a, 31b, and 31c, the light source control unit 38 performs an operation for turning off all the light sources 31a, 31b, and 31c and thereby stops emission of the R light, G light, and B light.

Note that when it is detected, based on the judgement result outputted from the judging unit 36, that something is wrong with at least one of the respective light sources 31a, 31b, and 31c, possible actions of the light source control unit 38 according to the present embodiment is not limited to performing an operation for turning off all and every one of the respective light sources 31a, 31b, and 31c, and include, for example, performing control to reduce the intensity of the pulsed light emitted by the at least one of the light sources to below the intensity value VS, performing control to reduce the intensity of the pulsed light emitted by each of the light sources to below the intensity value VS, and performing an operation for displaying a message or the like on the display device 4, indicating that something is wrong with the at least one of the light sources.

Also, the light source control unit 38 according to the present embodiment, for example, may detect whether or not there is anything wrong with the driving current supplied to each of the light sources 31 a, 31 b, and 31c based on the amperage outputted from the current monitoring unit 37 and stop supplying the driving current to each of the light sources when something wrong with the driving current is detected.

On the other hand, according to the present embodiment, for example, based on a response time from input of the driving currents for the light sources 31a, 31b, and 31c to emission of the pulsed light, the light source control unit 38 may detect whether or not there is any light source which exhibits disturbances in a waveform of pulsed light. A concrete operation and the like performed in such a case will be described below.

The clocking unit 35 operates, being timed with a synchronizing signal from the timing generator 28, thereby acquires a count value of a delay time RT which starts with a time point at which a driving current starts being supplied to the light source 31a, 31b, or 31c, and outputs the count value of the acquired delay time RT to the judging unit 36. Also, at the time point at which the pulse signal from the A/D converter 33 starts being inputted, the clocking unit 35 outputs the count value of the acquired delay time RT to the judging unit 36 and then resets the count value to 0.

That is, with the above-described operation of the clocking unit 35, a count value of a delay time RT1 between a time point at which the light source 31a starts emitting the R light and a time point at which the photodiode 27 starts outputting the pulse signal upon receiving the R light is outputted to the judging unit 36. Also, with the above-described operation of the clocking unit 35, a count value of a delay time RT2 between a time point at which the light source 31b starts emitting the G light and a time point at which the photodiode 27 starts outputting the pulse signal upon receiving the G light is outputted to the judging unit 36. Also, with the above-described operation of the clocking unit 35, a count value of a delay time RT3 between a time point at which the light source 31c starts emitting the B light and a time point at which the photodiode 27 starts outputting the pulse signal upon receiving the B light is outputted to the judging unit 36.

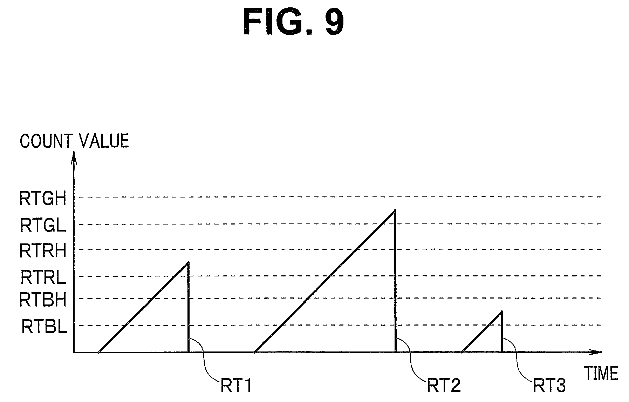

Based on the synchronizing signal from the timing generator 28, for example as shown in Fig. 9, the judging unit 36 detects each of: the count value of the delay time RT1 inputted being timed with the emission timing of the R light, the count value of the delay time RT2 inputted being timed with the emission timing of the G light, and the count value of the delay time RT3 inputted being timed with the emission timing of the B light. Fig. 9 is a diagram showing an example of time variation in count values of delay times.

Then, when it is detected that the count value of the delay time RT1 is within a range from a lower limit RTRL to an upper limit RTRH of a response time of the light source 31 a, that the count value of the delay time RT2 is within a range from a lower limit RTGL to an upper limit RTGH of a response time of the light source 31b, and that the count value of the delay time RT3 is within a range from a lower limit RTBL to an upper limit RTBH of a response time of the light source 31c, the judging unit 36 acquires a judgement result indicating that each of the light sources 31a, 31b, and 31 c is operating normally and outputs the acquired judgement result to the light source control unit 38.

On the other hand, when it is detected that the count value of the delay time RT1 is outside the range from the lower limit RTRL to the upper limit RTRH, the judging unit 36 acquires a judgement result indicating that something is wrong with the light source 31a and outputs the acquired judgement result to the light source control unit 38. Also, when it is detected that the count value of the delay time RT2 is outside the range from the lower limit RTGL to the upper limit RTGH, the judging unit 36 acquires a judgement result indicating that something is wrong with the light source 31b and outputs the acquired judgement result to the light source control unit 38. Also, when it is detected that the count value of the delay time RT3 is outside the range from the lower limit RTBL to the upper limit RTBH, the judging unit 36 acquires a judgement result indicating that something is wrong with the light source 31c and outputs the acquired judgement result to the light source control unit 38.

When it is detected, based on the judgement results outputted from the judging unit 36, that something is wrong with at least one of the light sources 31a, 31b, and 31c, the light source control unit 38 performs an operation for turning off all the light sources 31a, 31b, and 31c and thereby stops emission of the R light, G light, and B light.

As described above, the present embodiment can reliably detect whether or not there is anything wrong with a light source. Also, the present embodiment can prevent excessively strong light from being emitted from a light source with which something is wrong.

(Second Embodiment)

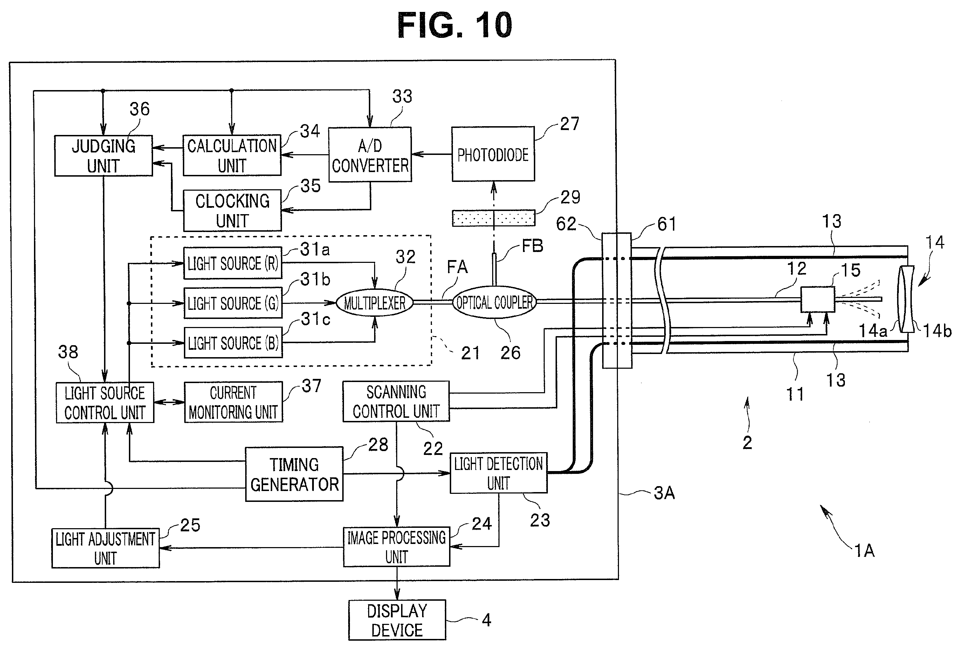

Figs. 10 to 13 relate to a second embodiment of the present invention. Fig. 10 is a diagram showing a configuration of principal part of an endoscope system according to the second embodiment.

Note that, in the present embodiment, detailed description of part similar in configuration and the like to the first embodiment will be omitted, and part different in configuration and the like from the first embodiment will be described mainly.

For example, as shown in Fig. 10, an endoscope system 1A includes an endoscope 2, a main apparatus 3A connectable with the endoscope 2, and a display device 4 connected to the main apparatus 3A.

The main apparatus 3A includes respective parts included in the main apparatus 3 and an optical filter 29 placed at a predetermined position on an optical path between the light source unit 21 and the photodiode 27.

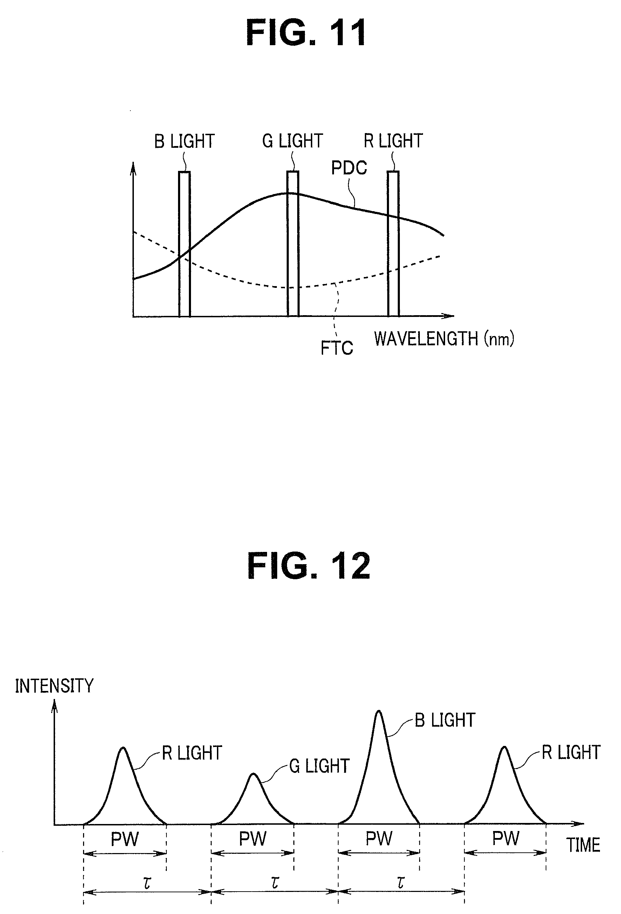

The optical filter 29 is placed, for example, on an optical path between the light emission surface of the optical fiber FB and photodiode 27 and is provided with transmission characteristics such as indicated by line segment FTC in Fig. 11. Specifically, the optical filter 29 is configured to have a transmittance of 1/Qr in the wavelength band of the R light, a transmittance of 1/Qg in the wavelength band of the G light, and a transmittance of 1/Qb in the wavelength band of the B light. Fig. 11 is a diagram showing an example of relationships among the wavelength bands of R light, G light, and B light, the spectral sensitivity characteristics of a photodiode, and the transmission characteristics of an optical filter.

That is, according to the present embodiment, under a condition of G=1, the threshold TH1 can be set so as to satisfy the relationship of Expression (5) above. Also, according to the present embodiment, under the condition of G=1, the threshold TH2 can be set so as to satisfy the relationship of Expression (10) above.

Next, operation of the present embodiment will be described.

After the main apparatus 3 is powered on, the light source control unit 38 operates, being timed with a synchronizing signal from the timing generator 28, and thereby performs an operation of switching the destination of the pulsed driving current such as shown in Fig. 3 every predetermined time $\tau$ in the order: light sources 31a -> 31b -> 31c -> 31a -> ... Then, the operation of the light source control unit 38 causes the R light, G light, and B light, which are pulsed lights such as shown in Fig. 4, to be emitted from the light emission surface of the optical fiber FB in this order every predetermined time $\tau$.

The photodiode 27 sequentially receives the R light, G light, and B light having intensities such as shown in Fig. 12, as lights which have passed through the optical filter 29. Then, the photodiode 27 generates an electrical signal according to the intensities of the R light, G light, and B light received sequentially, and outputs the electrical signal to the A/D converter 33. Fig. 12 is a diagram showing an example of pulsed lights which have passed through an optical filter having transmission characteristics such as shown in Fig. 11.

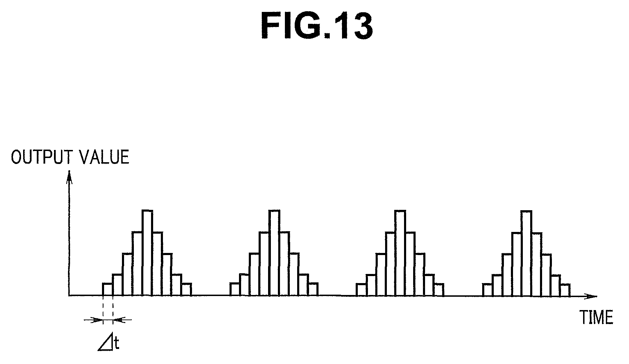

The A/D converter 33 operates being timed with a synchronizing signal from the timing generator 28, thereby converts the electrical signal outputted from the photodiode 27 into a pulse signal every sampling time $\Delta t$, which is shorter in duration than the pulse width PW, and outputs the pulse signal to the calculation unit 34 and clocking unit 35. Then, the operation of the A/D converter 33 causes a pulse signal with a waveform such as shown in Fig. 13 to be outputted to the calculation unit 34 and clocking unit 35, as a pulse signal according to intensities of the R light, G light, and B light received sequentially by the photodiode 27. Fig. 13 is a diagram showing an example of a pulse signal outputted from a photodiode which has received pulsed lights with intensities such as shown in Fig. 12.

Based on the synchronizing signal from the timing generator 28, the calculation unit 34 acquires an integrated value Spr by integrating output values of a pulse signal Sdr inputted being timed with the emission timing of the R light, and outputs the acquired integrated value Spr to the judging unit 36.

Based on the synchronizing signal from the timing generator 28, the calculation unit 34 acquires an integrated value Spg by integrating output values of a pulse signal Sdg inputted being timed with the emission timing of the G light, and outputs the acquired integrated value Spg to the judging unit 36.

Based on the synchronizing signal from the timing generator 28, the calculation unit 34 acquires an integrated value Spb by integrating output values of a pulse signal Sdb inputted being timed with the emission timing of the B light, and outputs the acquired integrated value Spb to the judging unit 36.

That is, with the above-mentioned calculation unit 34, a period during which the integrated value Spr is acquired and outputted to the judging unit 36, a period during which the integrated value Spg is acquired and outputted to the judging unit 36, and a period during which the integrated value Spb is acquired and outputted to the judging unit 36 are switched in this order every predetermined time $\tau$.

Also, the calculation unit 34 operates, being timed with a synchronizing signal from the timing generator 28, thereby acquires a total value SB, which is a sum total of the integrated values Spr, Spg, and Spb calculated within the base period BT longer in duration than the predetermined time $\tau$, and outputs the total value SB to the judging unit 36. Note that each time a new integrated value is calculated, the calculation unit 34 performs a process of acquiring the total value SB by overwriting the oldest of the respective integrated values calculated within the base period BT with the new integrated value. With this process, the total value SB outputted to the judging unit 36 is updated every predetermined time $\tau$.

The clocking unit 35 operates, being timed with a synchronizing signal from the timing generator 28, thereby acquires the count value of the elapsed time ET, and outputs the acquired count value of elapsed time ET to the judging unit 36.

Then, when it is detected that all the integrated values Spr, Spg, and Spb outputted from the calculation unit 34 are equal to or smaller than the threshold TH1, that the total value SB outputted from the calculation unit 34 is equal to or smaller than the threshold TH2, and that the count value of the elapsed time ET outputted from the clocking unit 35 is equal to or smaller than the threshold TH3, the judging unit 36 acquires a judgement result indicating that each of the light sources 31a, 31b, and 31c is operating normally and outputs the acquired judgement result to the light source control unit 38.

Also, when it is detected that any of the integrated values CrSpr, CgSpg, and CbSpb outputted from the calculation unit 34 exceeds the threshold TH1, that the total value SA outputted from the calculation unit 34 exceeds the threshold TH2, or that the count value of the elapsed time ET outputted from the clocking unit 35 exceeds the threshold TH3, the judging unit 36 acquires a judgement result indicating that something is wrong with at least one of the light sources 31a, 31b, and 31c, and outputs the acquired judgement result to the light source control unit 38.

When it is detected, based on the judgement result outputted from the judging unit 36, that each of the light sources 31a, 31 b, and 31c is operating normally, the light source control unit 38 continues the operation of switching the destination of the pulsed driving current shown in Fig. 3 every predetermined time $\tau$ in the order: light sources 31a -> 31b -> 31c -> 31a -> ...

Also, when it is detected, based on the judgement result outputted from the judging unit 36, that something is wrong with at least one of the light sources 31a, 31 b, and 31c, the light source control unit 38 performs an operation for turning off all the light sources 31 a, 31 b, and 31 c and thereby stops emission of the R light, G light, and B light.

As described above, the present embodiment can reliably detect whether or not there is anything wrong with a light source. Also, the present embodiment can prevent excessively strong light from being emitted from light sources with which something wrong is detected.

Note that the present invention is not limited to the two embodiments described above, and needless to say that various alterations and applications are possible without departing from the spirit of the invention.

The present application is based upon and claims the benefit of priority from Japanese Patent Application Laid-Open Publication No. 2014-114356, filed in Japan on June 2, 2014, the entire contents of which are incorporated in the specification, claims, and drawings herein by reference.

**Claims**

1. A lighting apparatus comprising:

    a light source unit provided with a first light source for emitting first light and a second light source for emitting second light in a wavelength band different from the first light and configured to emit the first light and the second light by sequentially switching between the first light and the second light;

    a light-receiving unit configured to receive the first light with first light-receiving sensitivity and output a first light detection signal and receive the second light with second light-receiving sensitivity different from the first light-receiving sensitivity and output a second light detection signal;

    a multiplication unit configured to generate a first multiplication signal and a second multiplication signal, where the first multiplication signal is obtained by multiplying the first light detection signal by a coefficient set according to the first light-receiving sensitivity and the second multiplication signal is obtained by multiplying the second light detection signal by a coefficient set according to the second light-receiving sensitivity;

    an integration unit configured to integrate at least one of the first multiplication signal and the second multiplication signal for a predetermined period, the first and second multiplication signals being generated by the multiplication unit;

    a judging unit configured to judge whether or not integration results produced by the integration unit exceed a threshold for judging a condition of the light source unit; and

    a control unit configured to control at least one of the first light source and the second light source based on a judgement result produced by the judging unit.

2. The lighting apparatus according to claim 1, wherein:

    the integration unit integrates the first multiplication signal over the predetermined period in which the first multiplication signal is inputted and integrates the second multiplication signal over the predetermined period in which the second multiplication signal is inputted; and

    the judging unit judges whether or not an integration result of the first multiplication signal and an integration result of the second multiplication signal each exceed the threshold.

3. The lighting apparatus according to claim 2, wherein the threshold is set based on a predetermined intensity value used to specify upper limit values of emission intensities of the first light and the second light.

4. The lighting apparatus according to claim 1, wherein the integration unit integrates the first multiplication signal and the second multiplication signal over the predetermined period longer than a switching time taken to switch between the first light and the second light.

**5.** The lighting apparatus according to claim 4, wherein the threshold is set based on a predetermined intensity value used to specify upper limit values of emission intensities of the first light and the second light, and on a total number of times the first light and the second light are received by the light-receiving unit during the predetermined period.

**6.** The lighting apparatus according to claim 2, wherein:

the integration unit further integrates the first multiplication signal and the second multiplication signal over a period longer than a switching time taken to switch between the first light and the second light; and
the judging unit makes threshold checks on each of the integration result of the first multiplication signal, the integration result of the second multiplication signal, and an integration result of the first multiplication signal and the second multiplication signal.

**7.** The lighting apparatus according to claim 6, wherein the judging unit makes threshold checks on each of the integration result of the first multiplication signal, the integration result of the second multiplication signal, the integration result of the first multiplication signal and the second multiplication signal, and an elapsed time from a time point at which a light detection signal starts being outputted from the light-receiving unit to a time point at which the light detection signal stops being outputted.

**8.** The lighting apparatus according to claim 6, wherein the judging unit makes threshold checks on each of the integration result of the first multiplication signal, the integration result of the second multiplication signal, the integration result of the first multiplication signal and the second multiplication signal, a first delay time from a time point at which the first light starts being emitted to a time point at which the first light detection signal starts being outputted, and a second delay time from a time point at which the second light starts being emitted to a time point at which the second light detection signal starts being outputted.

**9.** The lighting apparatus according to claim 1, wherein based on the judgement result produced by the judging unit, the control unit performs control to stop emission of the first light and the second light.

**10.** The lighting apparatus according to claim 3, wherein based on the judgement result produced by the judging unit, the control unit decreases intensity of light emitted from at least one of the first light source and the second light source to below the predetermined intensity value.

**11.** A lighting apparatus comprising:

a light source unit provided with a first light source for emitting first light and a second light source for emitting second light in a wavelength band different from the first light and configured to emit the first light and the second light by sequentially switching between the first light and the second light;
a light-receiving unit configured to receive the first light with first light-receiving sensitivity and output a first light detection signal and receive the second light with second light-receiving sensitivity different from the first light-receiving sensitivity and output a second light detection signal;
an optical filter placed at a predetermined position on an optical path between the light source unit and the light-receiving unit and configured to cause the first light emitted from the light source unit to be transmitted at first transmittance corresponding to an inverse of the first light-receiving sensitivity and cause the second light emitted from the light source unit to be transmitted at second transmittance corresponding to an inverse of the second light-receiving sensitivity;
an integration unit configured to integrate at least one of the first light detection signal and the second light detection signal outputted from the light-receiving unit for a predetermined period;
a judging unit configured to judge whether or not integration results produced by the integration unit exceed a threshold for judging a condition of the light source unit; and
a control unit configured to control at least one of the first light source and the second light source based on a judgement result produced by the judging unit.

**12.** The lighting apparatus according to claim 11, wherein
the integration unit integrates the first multiplication signal over the predetermined period in which the first light detection signal is inputted and integrates the second multiplication signal over the predetermined period in which the second light detection signal is inputted; and
the judging unit judges whether or not an integration result of the first light detection signal and an integration result of the second light detection signal each exceed the threshold.

**13.** The lighting apparatus according to claim 12, wherein the threshold is set based on a predetermined intensity value used to specify upper limit values of emission intensities of the first light and the second light.

**14.** The lighting apparatus according to claim 11, wherein the integration unit integrates the first light detection signal and the second light detection signal over the predetermined period longer than a switching time taken to switch between the first light and the second light.

**15.** The lighting apparatus according to claim 14, wherein the threshold is set based on a predetermined intensity value used to specify upper limit values of emission intensities of the first light and the second light, and on a total number of times the first light and the second light are received by the light-receiving unit during the predetermined period.

**16.** The lighting apparatus according to claim 12, wherein the integration unit further integrates the first light detection signal and the second light detection signal over a period longer than a switching time taken to switch between the first light and the second light; and

the judging unit makes threshold checks on each of the integration result of the first light detection signal, the integration result of the second light detection signal, and an integration result of the first light detection signal and the second light detection signal.

**17.** The lighting apparatus according to claim 16, wherein the judging unit makes threshold checks on each of the integration result of the first light detection signal, the integration result of the second light detection signal, the integration result of the first light detection signal and the second light detection signal, and an elapsed time from a time point at which a light detection signal starts being outputted from the light-receiving unit to a time point at which the light detection signal stops being outputted.

**18.** The lighting apparatus according to claim 16, wherein the judging unit makes threshold checks on each of the integration result of the first light detection signal, the integration result of the second light detection signal, the integration result of the first light detection signal and the second light detection signal, a first delay time from a time point at which the first light starts being emitted to a time point at which the first light detection signal starts being outputted, and a second delay time from a time point at which the second light starts being emitted to a time point at which the second light detection signal starts being outputted.

**19.** The lighting apparatus according to claim 11, wherein based on the judgement result produced by the judging unit, the control unit performs control to stop emission of the first light and the second light.

**20.** The lighting apparatus according to claim 13, wherein based on the judgement result produced by the judging unit, the control unit decreases intensity of light emitted from at least one of the first light source and the second light source to below the predetermined intensity value.

# FIG. 1

EP 3 081 143 A1

# FIG. 2

# FIG. 3

# FIG. 4

# FIG. 5

OUTPUT VALUE

TIME

$\Delta t$

# FIG. 6

INTEGRATED VALUE

CrSpr  CgSpg  CbSpb  CrSpr

TH1

$\tau$  $\tau$  $\tau$  TIME

# FIG. 7

TOTAL VALUE

TH2

SA

τ τ τ τ τ ...... τ τ τ τ τ τ τ τ τ τ   TIME

BT

# FIG. 8

COUNT VALUE

ET        ET        ET        ET

TH3

時間

# FIG. 9

COUNT VALUE

RTGH
RTGL
RTRH
RTRL
RTBH
RTBL

RT1  RT2  RT3  TIME

FIG. 10

# FIG. 11

B LIGHT    G LIGHT    R LIGHT
PDC

FTC    WAVELENGTH (nm)

# FIG. 12

INTENSITY

R LIGHT

G LIGHT

B LIGHT

R LIGHT

PW    PW    PW    PW    TIME

$\tau$    $\tau$    $\tau$

# FIG.13

OUTPUT VALUE

TIME

$\triangle$t

## INTERNATIONAL SEARCH REPORT

| International application No. |
|---|
| PCT/JP2015/056052 |

| A. CLASSIFICATION OF SUBJECT MATTER |
|---|
| *A61B1/00*(2006.01)i, *A61B1/06*(2006.01)i |

According to International Patent Classification (IPC) or to both national classification and IPC

| B. FIELDS SEARCHED |
|---|

Minimum documentation searched (classification system followed by classification symbols)
A61B1/00, A61B1/06

Documentation searched other than minimum documentation to the extent that such documents are included in the fields searched
Jitsuyo Shinan Koho          1922–1996   Jitsuyo Shinan Toroku Koho   1996–2015
Kokai Jitsuyo Shinan Koho   1971–2015   Toroku Jitsuyo Shinan Koho   1994–2015

Electronic data base consulted during the international search (name of data base and, where practicable, search terms used)

| C. DOCUMENTS CONSIDERED TO BE RELEVANT |
|---|

| Category* | Citation of document, with indication, where appropriate, of the relevant passages | Relevant to claim No. |
|---|---|---|
| A | JP 5467181 B1   (Olympus Medical Systems Corp.), 09 April 2014 (09.04.2014), paragraph [0030] & US 2014/49624 A1        & EP 2737843 A1 & CN 103796571 A | 1-20 |
| A | JP 2014-301 A  (Fujifilm Corp.), 09 January 2014 (09.01.2014), paragraphs [0034], [0035] & US 2013/345517 A1      & CN 103505174 A | 1-20 |

☐ Further documents are listed in the continuation of Box C.      ☐ See patent family annex.

| | |
|---|---|
| *     Special categories of cited documents: "A"   document defining the general state of the art which is not considered    to be of particular relevance "E"   earlier application or patent but published on or after the international filing date "L"   document which may throw doubts on priority claim(s) or which is cited to establish the publication date of another citation or other special reason (as specified) "O"   document referring to an oral disclosure, use, exhibition or other means "P"   document published prior to the international filing date but later than the priority date claimed | "T"   later document published after the international filing date or priority date and not in conflict with the application but cited to understand the principle or theory underlying the invention "X"   document of particular relevance; the claimed invention cannot be considered novel or cannot be considered to involve an inventive step when the document is taken alone "Y"   document of particular relevance; the claimed invention cannot be considered to involve an inventive step when the document is combined with one or more other such documents, such combination being obvious to a person skilled in the art "&"   document member of the same patent family |

| Date of the actual completion of the international search 01 May 2015 (01.05.15) | Date of mailing of the international search report 19 May 2015 (19.05.15) |
|---|---|
| Name and mailing address of the ISA/ Japan Patent Office 3-4-3,Kasumigaseki,Chiyoda-ku, Tokyo 100-8915,Japan | Authorized officer Telephone No. |

Form PCT/ISA/210 (second sheet) (July 2009)

23

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5363688 B
- JP 2014114356 A